Europäisches Patentamt

**European Patent Office**

**Office européen des brevets**

(19)

(11) Numéro de publication: **0 289 391**
**B1**

(12)   **FASCICULE DE BREVET EUROPEEN**

(45) Date de publication du fascicule du brevet:
03.10.90

(51) Int. Cl.⁵: **C07C 2/76, B01J 19/00**

(21) Numéro de dépôt: **88400941.6**

(22) Date de dépôt: **19.04.88**

(54)   Procédé de conversion/thermique du méthane en hydrocarbures de poids moléculaires plus élevés, réacteur pour la mise en oeuvre du procédé et procédé de réalisation du réacteur.

(30) Priorité: **28.04.87 FR 8706064**

(43) Date de publication de la demande:
**02.11.88 Bulletin 88/44**

(45) Mention de la délivrance du brevet:
**03.10.90 Bulletin 90/40**

(84) Etats contractants désignés:
**DE GB IT NL**

(56) Documents cités:
**WO-A-87/00546**
**DE-A-1 542 406**
**FR-A-711 394**
**FR-A-1 364 835**
**FR-A-2 589 859**

(73) Titulaire: **INSTITUT FRANCAIS DU PETROLE, 4, Avenue de Bois-Préau, F-92502 Rueil-Malmaison(FR)**

(72) Inventeur: **Alagy, Jacques, 24, Chemin Beckensteiner, F-69260 Charbonnieres(FR)**
Inventeur: **Busson, Christian, 11, Chemin du Cogny, F-69570 Dardilly(FR)**
Inventeur: **Fouquet, Michel, 3, Allée de la Malletière, F-69600 Oullins(FR)**

## Description

L'invention concerne un procédé amélioré de conversion thermique du méthane en hydrocarbures de poids moléculaires plus élevés, le réacteur pour la mise en œuvre de ce procédé et le procédé de réalisation du réacteur.

Parmi les sources de méthane se trouvent les gaz naturels et les gaz de raffinerie. Les gaz naturels peuvent être ou non des gaz associés à l'huile brute; leur composition varie de façon assez sensible suivant leur provenance, mails ils contiennent généralement un pourcentage volumique de méthane compris entre 60 et 95%. Ce méthane est toujours associé à d'autres alcanes supérieurs, pouvant atteindre et même dépasser des hydrocarbures en $C_6$. Divers procédés cryogéniques permettent de séparer en plusieurs fractions les gaz une fois débarassés de l'eau et des composants acides: azote, gaz naturels liquéfiés dont on sépare la fraction propane et butane, et une fraction composée essentiellement de méthane associé à une faible quantité d'éthane. Cette dernière fraction est soit réinjectée dans le puits pour maintenir la pression qui fait monter le pétrole brut, soit expédiée par gazoduc comme gaz combustible, soit encore brûlée à la torche.

D'autres sources de méthane sont les gaz de raffinerie qui sont d'origines multiple: gaz de première distillation du pétrole brut, gaz d'hydroréformage, d'hydrotraitements divers, gaz de craquage thermique, gaz de craquage catalytique; tous ces gaz contiennent, dans des proportions diverses, du méthane associé à de nombreux autres composants gazeux, tels que des hydrocarbures légers, de l'azote, du $C_2$, de l'hydrogène, etc...

C'est ainsi, par exemple qu'un gaz effluent d'une unité de craquage catalytique en lit fluidisé comprend, après lavage, de l'ordre de 30% en volume de méthane. Ce gaz est souvent fractionné par refroidissement sous pression, permettant d'obtenir deux fractions, l'une comprenant de l'hydrogène, de l'azote, du méthane et une faible partie d'éthylène, l'autre fraction étant composée de la majeure partie de l'éthylène initial, d'éthane, de propane et de propylène. Cette dernière fraction peut être avantageusement envoyée à une unité de type DIMERSOL, alors que la première est retournée vers le réseau de fuel-gaz de la raffinerie où elle est utilisée comme combustible.

La conversion de méthane en hydrocarbures de poids moléculaires plus élevés présente donc un intérêt certain; c'est ainsi que, dans les gisements lointains de gaz naturels ou de gaz associés, la conversion du méthane en acétylène, éthylène et aromatiques peut permettre, par l'utilisation d'enchainements de procédés connus, d'obtenir des fractions liquides plus facilement transportables et/ou valorisables.

Par exemple, après séparation des solides formés éventuellement, on peut séparer la fraction de composés aromatiques, puis traiter la fraction gazeuse tout d'abord dans des unités permettant l'oligomérisation et/ou la cyclisation de l'acétylène, et ensuite, après une nouvelle séparation gaz/liquides, traiter la fraction gazeuse résiduelle, riche en éthylène, dans des unités de type Dimersol, qui permettent d'obtenir des oligomères de l'éthylène.

Sur les lieux même de raffinage, la conversion, même partielle, du méthane en produits plus facilement valorisables présente également un grand intérêt économique.

Il a déjà été proposé différentes méthodes de conversion du méthane; c'est ainsi que le brevet US 4 199 533 décrit une méthode d'obtention d'éthylène et/ou d'éthane à partir de méthane, consistant à faire réagir sur ce produit du chlore à température supérieure à 700°C. Ce procédé présente l'inconvénient important de mise en œuvre à haute température de gaz très corrosifs, comme le chlore et l'acide chlorhydrique.

De plus, le brevet FR-A 711 394 décrit un procédé de transformation du méthane où l'on tire la chaleur nécessaire au chauffage des points de départ d'un procédé de production de gaz. En outre, le brevet FR-A 1 364 835 décrit un procédé empêchant les réactions secondaires dans l'oxydation des hydrocarbures. Par ailleurs, l'art antérieur, peut être illustré par les brevets WO-A 8 700 546, DE-A 1 542 406 et notamment par le brevet FR -A 2 589 859 qui décrit un procédé de conversion thermique du méthane dans une zone continue en matière céramique comprenant une pluralité de canaux juxtaposés. Dans un premier type de canaux, circule la charge tandis que dans un deuxième type de canaux circule un fluide caloporteur.

De nombreux procédés de craquage catalytique du méthane ont été décrits dans l'art antérieur, en utilisant par exemple des catalyseurs de type zéolithique, comme dans le brevet EP 93 543, mais tous les catalyseurs utilisés présentent une durée de vie très courte, due aux dépôts de coke formés dans la réaction.

Par ailleurs, le couplage oxydant du méthane est un procédé bien connu, pouvant être conduit soit en présence d'oxygène, soit même en l'absence d'oxygène, des oxydes métalliques intervenant alors dans la réaction en étant réduits; on peut citer, à titre d'exemples de ces procédés les brevets US 4 172 810, 4 239 658, 4 443 644 et 4 444 984; ces procédés sont discontinus, puisque l'oxyde métallique doit être régénéré.

Parmi les procédés de craquage thermique susceptibles de transformer du méthane, le procédé dit "procédé Wulff", consiste à utiliser des masses de contact réfractaires; dans un premier temps on chauffe la masse réfractaire par combustion à l'air d'un combustible qui peut être constitué par la charge elle-même puis, dans un second temps, l'hydrocarbure à craquer est décomposé en absorbant de la chaleur emmagasinée par le matériau réfractaire lors de la période précédente; il s'agit donc d'un procédé discontinu.

Les procédés à arc électrique et à plasma sont axés essentiellement sur la préparation d'acétylène ; leur consommation élevée en énergie électrique les rend difficilement exploitables.

Un autre type de procédé, quelquefois nommé autothermique, consiste à brûler une partie de la charge pour fournir les calories nécessaires à la réaction de craquage ; ce type de procédé met en oeuvre un brûleur dans lequel environ 1/3 de l'hydrocarbure est brûlé, le reste étant craqué. Etant donné les hauts niveaux thermiques atteints, ce type de procédé produit essentiellement de l'acétylène et du coke.

Le brevet FR. 1211.695 décrit un procédé de pyrolyse combiné d'hydrocarbures consistant à mélanger du méthane à des gaz de combustion chauds ne comportant pas d'oxygène en excès, puis à injecter dans le mélange obtenu des hydrocarbures paraffiniques à plus d'un atome de carbone ; suivant ce procédé, une très faible partie du méthane peut être transformée en acétylène.

Le couplage thermique deshydrogénant du méthane est fortement endothermique et nécessite l'obtention, à un niveau de température élevé, de l'ordre de 1100 à 1500 °C, d'une densité de flux thermique très importante. Il est nécessaire que l'apport maximum de chaleur soit effectué dans la zone où s'effectuent les réactions endothermiques de craquage et de deshydrogénation ; par ailleurs, l'obtention de produits valorisables, tels qu'acétylène, éthylène et/ou composés aromatiques, nécessite un temps de contact très court suivi d'une trempe rapide, de façon à obtenir un profil de température de type "carré".

Il n'existe pas, actuellement de procédé industriel utilisant un transfert de chaleur contrôlé à travers une paroi qui permette de transformer le méthane en hydrocarbures facilement valorisables.

L'objet de cette invention est de pallier cette lacune en proposant un procédé de conversion thermique du méthane en hydrocarbures de poids moléculaire plus élevé, ainsi qu'un réacteur pour la mise en oeuvre de ce procédé conduisant à une mise en oeuvre plus facile, plus souple, et mieux contrôlée.

Plus particulièrement, l'invention concerne un procédé de conversion thermique du méthane en hydrocarbures de poids moléculaires plus élevés dans une zone de réaction, en matière céramique, comprenant une série de canaux juxtaposés groupés par rangées et s'étendant sur une partie au moins de la longueur de la dite zone de réaction, parallèlement à son axe, les dites rangées de canaux étant non adjacentes les unes par rapport aux autres, la zone de réaction comprenant en outre d'une part une zone de chauffage entourant sensiblement les dites rangées de canaux soit sur la dite partie de la zone de réaction soit sur une partie de la longueur de la dite zone de réaction quand les dits canaux s'étendent sur toute la longueur de la zone de réaction, d'autre part une zone de refroidissement faisant suite à la dite zone de chauffage, caractérisé en ce que l'on fait circuler un gaz renfermant du méthane, par exemple un pourcentage molaire de méthane compris entre 10 et 99 % dans les canaux des dites rangées, en ce que l'on chauffe la zone de chauffage par apport d'énergie électrique par sections transversales successives indépendantes, sensiblement perpendiculaires à l'axe de la dite zone de réaction, sensiblement parallèles au plan desdites rangées et étanches vis-à-vis des canaux de la zone de réaction, en ce que l'on introduit un fluide de refroidissement dans la dite zone de refrcidissement et en ce que l'on recueille les dits hydrocarbures de poids moléculaires plus élevés à l'extremité de la zone réactionnelle.

Par zone de réaction ou zone réactionnelle, on entend la zone de chauffage qui chauffe les mélanges gazeux constituant les charges ainsi que la zone de refroidissement (ou zone de trempe) qui refroidit les effluents soit indirectement par l'intermédiaire du contact d'un fluide froid sur les parois des canaux, soit directement par mélange des effluents et d'un fluide froid.

De manière plus particulière, on chauffe la dite zone de chauffage par apport d'énergie électrique, à travers des moyens de chauffage tels que des résistances électriques, par exemple, chauffant par effet Joule les parois des rangées de canaux où circulent les mélanges gazeux.

Ce type de chauffage statique, par opposition à un chauffage dynamique par circulation d'un fluide caloporteur autour des canaux conduisant le mélange gazeux réactionnel, contribue à une mise en oeuvre du procédé plus facile et surtout mieux contrôlée.

De ce fait, le taux de conversion du procédé se trouve augmenté et la sélectivité est améliorée.

Selon l'une des caractéristiques principales de l'invention, les résistances électriques qui fournissent de la chaleur à la zone de chauffage sont alimentées de façon indépendante en énergie électrique soit isolément soit par petits groupes, de façon à définir des sections de chauffage le long de la zone de chauffage et à pouvoir ainsi moduler la quantité d'énergie fournie tout au long de cette zone.

La zone de chauffage est généralement composée de 3 à 20 sections de chauffage, de préférence de 5 à 12. Dans la première partie de cette zone, le gaz renfermant du méthane, préalablement chauffé à 650 °C environ, est généralement porté rapidement à une température maximale au plus égale à 1500 °C, et avantageusement entre 1000 et 1300 °C. (Le début de la zone de chauffage est située à l'endroit où la charge est introduite).

Dans la deuxième partie de la zone de chauffage, faisant suite à la première, on module l'énergie électrique fournie à chaque section de chauffage de cette partie de manière à maintenir les gaz à une température sensiblement constante, voisine de la température maximale de la première partie, de façon à obtenir un profil de température pratiquement plat tout au long de cette deuxième partie.

La modulation de ces sections de chauffage est réalisée de façon classique ; les éléments résistants correspondant aux sections précitées sont généralement alimentés par des ensembles modulateurs à thyristors. Des transformateurs permettent éventuellement d'adapter les tensions à priori, alors que les modulateurs permettent le réglage fin et continu de la puissance injectée.

Pour permettre la régulation de l'ensemble, les canaux où circule la charge sont, par exemple, munis, au niveau de la zone de chauffage et de préférence au niveau de chauffage maximal, d'au moins une canne pyrométrique à thermocouple adaptée au niveau de température, tel par exemple que chromel-alumel ou Pt/Pt Rh. Ces thermocouples constituent des thermocouples de commande qui transmettent leurs informations au régulateur qui commande le modulateur à thyristor.

Dans la première partie de la zone de chauffage, l'énergie électrique sert presque exclusivement à porter le mélange réactionnel de sa température initiale - environ 650 °C - à la température de 1200 °C par exemple, vers laquelle les réactions endothermiques de couplage déshydrogénant du méthane ont lieu. C'est donc au début de la deuxième partie de la zone de chauffage qu'il est nécessaire de fournir l'énergie maximum au milieu réactionnel, ce qui est facilement réalisé par la modulation d'une ou plusieurs sections de chauffage.

La longueur de la première partie de la zone de chauffage représente généralement de 5 à 50 % de la longueur totale de la zone de chauffage, avantageusement de 10 à 30 %, et de manière préférée de 15 à 25 % de cette zone.

L'énergie électrique fournie à cette première partie de la zone de chauffage est telle qu'elle génère un fort gradient de température, compris généralement entre 5 et 60 °C/cm, et avantageusement entre 10 et 30 °C/cm.

Dans la deuxième partie de la zone de chauffage, on module l'énergie électrique fournie aux différentes sections de chauffage de cette zone de façon à ce que la variation de température tout au long de cette zone soit faible, généralement inférieure à environ 20 °C (+ ou - 10 °C autour de la valeur de consigne) et avantageusement inférieure à environ 10 °C.

La longueur de la zone de chauffage est généralement comprise entre 50 et 90 % de la longueur de la zone réactionnelle totale.

On obtient notamment dans les conditions de chauffage ci-dessus, un flux thermique très important à un niveau de température élevé.

L'espace défini entre les rangées de canaux où circule la charge, peut être occupé par une seconde série de canaux déterminant également au moins une rangée de canaux en alternance avec ceux où circule le mélange gazeux. Ces canaux sont généralement divisés en au moins deux parties non communicantes entre elles et non communicantes avec les canaux de circulation de la charge.

Selon un premier mode de réalisation de la zone de chauffage, l'espace dans le réacteur entre plaques réservées à la circulation de la charge peut être rempli par au moins une plaque en général sensiblement identique à la plaque de canaux de circulation, dont les canaux de deuxième série sont disposés sensiblement perpendiculairement aux canaux de circulation de la charge. A l'intérieur des canaux de deuxième série sont logés les éléments de chauffage.

Selon un autre mode de réalisation de la zone de chauffage, les canaux de deuxième série peuvent être sensiblement parallèles aux canaux de circulation. Les moyens de chauffage sont alors disposés de façon sensiblement parallèle au plan des plaques des canaux de circulation mais dans une direction sensiblement perpendiculaire à l'axe des canaux. On creuse à cet effet les parois latérales de la deuxième série de canaux de façon à permettre leur passage et à déterminer des sections de chauffage indépendantes et sensiblement perpendiculaires à la direction de l'écoulement de la charge.

De préférence, les plaques unitaires servant à conduire les gaz du procédé sont identiques entre elles. Il est également préférable que les plaques dans lesquelles sont disposés les éléments de chauffage soient similaires entre elles ; elles peuvent par contre être différentes des plaques conduisant les gaz.

La zone de chauffage est suivie d'une zone de refroidissement (ou trempe) de façon à abaisser très rapidement la température des effluents de la zone de chauffage vers environ 300°C par exemple.

Selon un mode de réalisation, avec une trempe indirecte, les canaux où circule la charge s'étendent en général sur toute la longueur de la zone de réaction. Les canaux de deuxième série, de longueur sensiblement égale aux premiers, sont divisés en au moins deux parties non communicantes entre elles et non communicantes avec les canaux de circulation de la charge. La première partie (côté alimentation) correspond à la zone de chauffage tandis que la seconde partie (côté sortie de l'effluent) comprend la zone de refroidissement ou de trempe où l'on fait s'écouler, en général parallèlement à l'écoulement de la charge, un fluide réfrigérant.

Dans le cadre de l'invention, on peut réaliser l'ensemble continu réacteur de chauffage et échangeur de trempe soit sous forme d'un monobloc, soit encore par juxtaposition jointive d'éléments unitaires, de forme identique, qui sont assemblés entre eux par tout moyen adéquat, comme par exemple à l'aide de brides. L'utilisation de céramique résistant à des températures supérieures à 1150 °C, et plus particulièrement du carbure de silicium, matériau facilement extrudable, rend facile la mise en oeuvre de tels ensembles ou éléments d'ensembles.

Dans un mode de réalisation de l'invention, l'arrivée du fluide de refroidissement dans les rangées de canaux constituant la seconde section et destinés à le véhiculer s'effectue sensiblement perpendiculairement à l'axe de ces rangées de canaux, au moyen d'une ouverture pratiquée dans l'une des parois latérales des canaux concernés situés à la périphérie, et les canaux d'une même rangée sont mis en communication au niveau de l'arrivée du fluide réfrigérant par des ouvertures pratiquées dans leurs parois latérales, de façon à ce que la totalité des canaux destinés à cet usage soit parcourue par le fluide.

De nombreux échangeurs indirects de chaleur en céramique ont été décrits dans l'art antérieur ; leur ·

domaine d'utilisation concerne essentiellement les moteurs à turbine, où le matériau de l'échangeur doit résister à des températures de l'ordre de 1200 à 1400 °C. On peut citer, par exemple, le brevet français 2.436.956, le brevet US 4.421.702, les demandes de brevets japonais 59.046.496 et 59.050.082, ainsi que les brevets français 2.414.988 et 2.436.958 (brevet d'addition du précédent), ce dernier brevet décrivant un procédé de fabrication d'un élément d'échange indirect de chaleur en matière céramique pouvant être avantageusement utilisé dans le cadre de l'invention, moyennant des aménagements.

Les fluides utilisables pour refroidir les effluents au niveau de la zone de trempe indirecte peuvent être par exemple de l'air, seul ou en mélange avec des fumées de combustion, ou encore de la vapeur d'eau à basse température et sous basse pression.

Selon un autre mode de réalisation, dans le cas d'une réaction avec trempe directe, les canaux où circule la charge s'étendent en général sur une partie de la longueur de la zone réactionnelle et l'espace défini entre les rangées de ces canaux est réservé à la zone de chauffage dont la longueur est sensiblement égale à la longueur des canaux où circule la charge.

Les effluents de réaction quittant la zone de chauffage sont très rapidement refroidis par injection et mise en contact directe dans ces effluents, par exemple à l'aide d'au moins un injecteur en matériau céramique disposé à la périphérie du réacteur, des fluides de refroidissement, tels que des gaz de pétrole liquéfiés, du propane ou des huiles hydrocarbonées, le propane étant le gaz de trempe préféré car , par ailleurs, il peut être également partiellement craqué et il peut ainsi contribuer à la formation de produits tels que l'éthylène.

L'utilisation de matériaux céramiques, et de préférence du carbure de silicium permet des températures de parois pouvant atteindre 1500 °C en utilisation continue, ce qui dépasse les limites de la métallurgie actuelle et permet d'augmenter la densité du flux thermique et la température de mise en oeuvre de la réaction.

Par ailleurs, l'utilisation de différentes sections transversales de chauffage, indépendantes les unes des autres, au niveau de la deuxième partie de la zone de chauffage, permet d'apporter le maximum d'énergie thermique à l'endroit où s'effectue la plus grande partie des réactions de craquage endothermiques, et de maintenir dans le reste de la zone de chauffage une température quasi uniforme.

Ces caractéristiques, alliées à la grande valeur du rapport de la surface d'échange (S) au volume réactionnel (V), généralement compris entre 200 et 1000 $m^{-1}$ , permettent d'obtenir, grâce à ce procédé une conversion thermique du méthane en acétylène, éthylène et produits benzéniques s'effectuant avec un bon taux de conversion et une sélectivité élevée.

Les charges hydrocarbonées utilisables dans le cadre de l'invention sont des charges gazeuses dans les conditions normales de température et de pression, comprenant habituellement un pourcentage molaire de méthane compris entre 10 et 99 %, par exemple entre 20 et 99 % et préférentiellement entre 30 et 80 %.

Le reste de la charge peut être constitué par des hydrocarbures aliphatiques, saturés ou non, comprenant un nombre d'atomes de carbone égal ou supérieur à deux, tels que par exemple, l'éthylène, l'éthane, le propane ou le propylène ; d'autres éléments gazeux constitutifs de la charge peuvent être l'azote, le gaz carbonique ou l'oxyde de carbone ou, de préférence l'hydrogène, dont la présence permet de réduire la formation de coke. La proportion molaire d'hydrogène peut être de 1 à 90 %.

On peut, en restant dans le cadre de l'invention, rajouter aux charges définies ci-dessus de la vapeur d'eau de dilution ; le rapport pondéral de la vapeur d'eau de dilution à la charge hydrocarbonée est de l'ordre de 0,1 à 1.

Les charges à traiter ont un temps de séjour dans la zone réactionnelle généralement compris entre 2 et 1000 millisecondes et de préférence entre 30 et 300 millisecondes.

L'invention concerne aussi le dispositif pour la mise en oeuvre du procédé. Ce dispositif peut être également utilisable pour tout procédé de vapocraquage d'un hydrocarbure contenant au moins deux atomes de carbone.

Plus particulièrement, l'invention concerne un dispositif comprenant des moyens d'alimentation en mélange gazeux et des moyens d'évacuation des effluents produits, caractérisé en ce qu'il comprend un réacteur 1 de forme allongée, de préférence à section carrée ou rectangulaire, en matière céramique, ayant un axe de symétrie et relié d'une part, à une première extrémité, aux dits moyens d'alimentation et d'autre part, à l'extrémité opposée, aux dits moyens d'évacuation, le dit réacteur comprenant une série de canaux 11 juxtaposés, groupés par rangées, adaptés à la circulation du mélange gazeux et s'étendant sur une partie au moins de la longueur du dit réacteur parallèlement à son axe, les dites rangées de canaux formant une pluralité de plaques 4 non adjacentes les unes par rapport aux autres et définissant des espaces 17 étanches entre les dites plaques, le dit réacteur comportant en outre sur une première zone (côté première extrémité) des moyens de chauffage électrique 5 dans chacun des dits espaces adaptés à chauffer les dites plaques de canaux par sections transversales successives, indépendantes, sensiblement perpendiculaires à l'axe du dit réacteur et sensiblement parallèles au plan des dites plaques, les dits moyens de chauffage entourant ainsi sensiblement lesdites plaques 4 de canaux soit sur la dite partie du réacteur, soit sur une partie de la longueur du dit réacteur 1 quand les dits canaux 11 s'étendent sur toute la longueur du réacteur, le dit réacteur comportant en outre des moyens d'asservissement et de modulation 7, 8 de chauffage reliés aux dits moyens de chauffage, le dit réacteur comportant par ailleurs sur une deuxième zone (côté extrémité opposée) contiguë à la première et non communicante avec la première zone, des moyens de refroidissement des effluents adaptés à refroidir, soit par circulation d'un fluide froid dans les dits espaces étanches de la deuxième zone du réacteur, les plaques 4 de canaux (trempe indirecte), soit par contact direct, les effluents quittant les dits canaux 11 (trempe directe).

Chacune de ces plaques forme en général au moins une rangée de canaux unitaires mais selon un mode particulier de réalisation du procédé chaque canal unitaire peut être subdivisé en une pluralité de canaux élémentaires plus petits.

Le nombre total de rangées de canaux n'est pas déterminant dans le procédé; il est évidemment fonction de la dimension de l'ensemble réacteur de chauffage et dispositif de trempe et des dimensions d'un canal unitaire. Cependant, les deux rangées de canaux ou d'espaces extérieurs sont, dans le cadre de l'invention, de préférence occupées par les moyens de chauffage ou de refroidissement.

Le nombre de canaux unitaires par rangée n'est pas non plus déterminant et est fonction de la dimension globale de l'ensemble et de la dimension d'un canal unitaire.

Un canal unitaire présente une section généralement comprise entre 9 et 900 mm² et avantageusement comprise entre 25 et 100 mm². Sa section peut être de forme quelconque mais est préférentiellement carrée ou rectangulaire. La longueur d'un canal peut être variable suivant les charges à traiter, la température du procédé, le temps de contact désiré. Les plaques unitaires sont généralement de forme parallélipipédique. Il est préférable, dans le cadre de l'invention que les plaques unitaires utilisées présentent la même géométrie et un nombre identique de canaux unitaires.

Tout matériau présentant une conductibilité électrique satisfaisante peut être utilisé sous forme de résistance électrique dans le cadre de l'invention, sous condition qu'il soit stable à une température de 1500 à 1600 °C, par exemple du molybdène et des alliages nickel - chrome.

Les plaques unitaires, ou rangées, sont constituées par un matériau réfractaire ; on peut utiliser des céramiques comme la mullite, la cordiérite, le carbure de silicium, les nitrures de silicium, la silice ou l'alumine ; le carbure de silicium est le matériau préférentiellement choisi car il présente une bonne conductivité thermique et se prête à l'extrusion.

La distance entre rangées de canaux (ou plaques) définit des espaces destinés à recevoir le chauffage ou le fluide de refroidissement le cas échéant, est généralement comprise entre 1 et 150 mm, et avantageusement entre 3 et 100 mm. La distance, selon l'axe de la zone réactionnelle, entre les divers éléments de chauffage électriques est généralement comprise entre 1 et 200 mm.

Pour une distance avantageusement comprise entre 3 et 50 mm, on a obtenu une très bonne stabilité de la température dans la zone de chauffage maximum (zone à température sensiblement constante).

L'invention concerne aussi un procédé de réalisation du dispositif en matière céramique pour la mise en œuvre du procédé.

On juxtapose dans une première zone du réacteur de manière sensiblement adjacente, successivement en alternance une plaque de canaux 11 d'une série et une plaque de canaux 28 d'une deuxième série, on détermine des sections de chauffage dans la dite plaque de canaux 28 en introduisant des moyens de chauffage 5 de manière sensiblement parallèle au plan des dites plaques soit dans la direction des canaux 28 de deuxième série soit dans une direction sensiblement perpendiculaire aux dits canaux 28, les dits moyens de chauffage 5 étant reliés aux dits moyens d'asservissement et de modulation du chauffage 7, 8, les dites plaques de canaux 11 et 28 étant juxtaposées de telle façon que les canaux 11 de première série soient sensiblement perpendiculaires aux dites sections; on isole, si nécessaire, de la dite plaque de canaux 11, la plaque contenant les dits moyens de chauffage; on introduit dans une deuxième zone du réacteur soit un moyen de refroidissement direct, de préférence au voisinage de la sortie des effluents des canaux 11 soit un moyen de refroidissement indirect des canaux 11 suivant lequel on juxtapose de manière alternée et sensiblement adjacente une plaque de canaux 11 adaptés à recevoir les effluents dans le prolongement des canaux 11 de la dite première zone et une plaque de canaux 18 sensiblement parallèles aux canaux 11 et adaptés à faire circuler un fluide de refroidissement; on isole si nécessaire la dite plaque de canaux 18 par un cloisonnement 32.

On établit enfin dans le cas du refroidissement indirect l'étanchéité entre les deux partie du réacteur par des moyens d'étanchéité tels que des brides en céramique.

L'utilisation de céramique et plus particulièrement du carbure de silicium, matériau facilement extrudable rend facile la réalisation de tels ensembles ou d'éléments d'ensemble.

L'invention sera mieux comprise au vu des figures représentant à titre illustratif et non limitatif divers modes de réalisation parmi lesquelles :

- la figure 1A représente une coupe longitudinale du réacteur avec trempe directe et la figure 1B une vue du réacteur avec trempe indirecte,
- la figure 2 représente une coupe transversale du réacteur selon un mode de réalisation particulier, et
- les figures 3 et 3A illustrent la zone de chauffage.

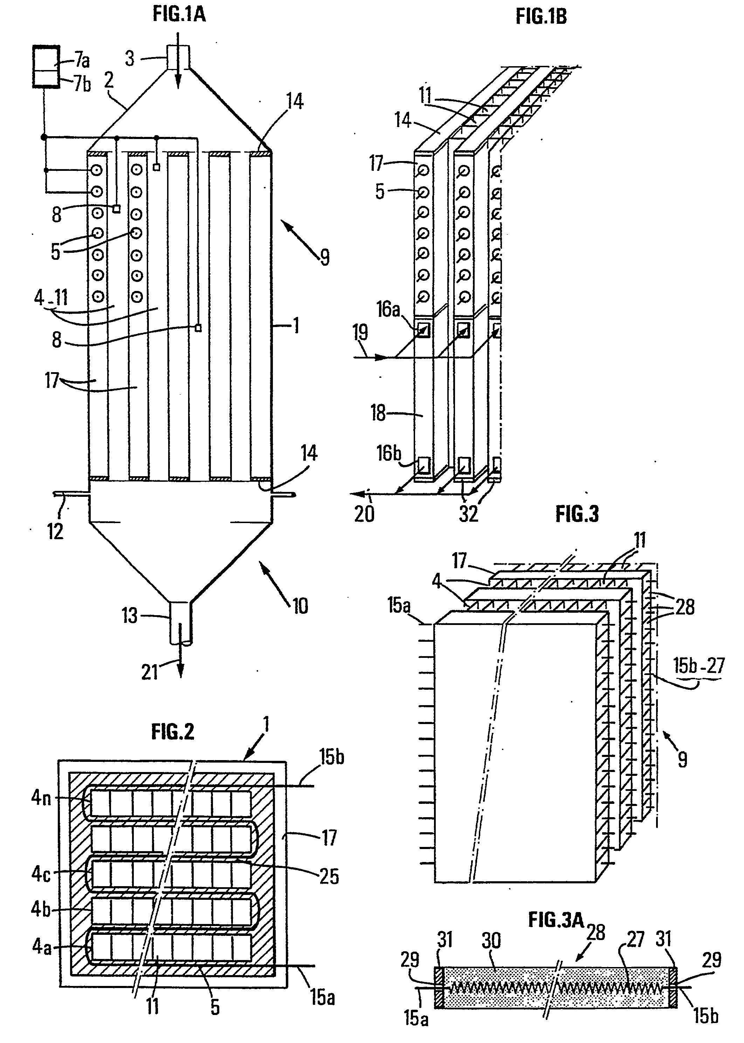

Sur la figure 1A, on a représenté, selon un mode de réalisation, un réacteur 1 ou zone réactionnelle à trempe directe, vertical, de forme allongée et de section carrée, comprenant un distributeur 2 permettant d'alimenter par un orifice d'entrée 3 le réacteur en mélange gazeux réactionnel. Ce dernier contient par exemple (50 %) de méthane et a été préchauffé dans une zone de préchauffage conventionnelle non représentée sur la figure, de préférence par convection. Le réacteur est de type multicanaux à section carrée. Il comprend une pluralité de plaques unitaires 4 parallèlipipédiques sensiblement parallèles entre elles et définissant des rangées de canaux 11 sensiblement parallèles entre eux par lesquels s'écoule le mélange gazeux réactionnel. Ces plaques de canaux sont en règle générale non adjacentes et définissent des espaces 17 dans lesquels vont se situer les moyens de chauffage 5 des résistances électriques par exemple, décrits ci-dessous, qui entourent sensiblement les diverses plaques de canaux et qui sont isolés de manière étanche par des cloisonnements 14, de la charge et des effluents réactionnels. La zone de chauffage 9 dans laquelle sont logés les moyens de chauffage 5 est agencée de façon à définir des sections de chauffage transversales et sensiblement perpendiculaires à l'axe du réacteur défini selon la direction d'écoulement de la charge (sections horizontales dans le cas de la figure 1). Elles sont généralement chauffées de manière indépendante et sont alimentées électriquement grâce à une paire d'électrodes (15a, 15b, Fig. 2).

La zone de chauffage 9, de longueur représentant par exemple 90% du réacteur est agencée de façon à ce que, dans sa première partie (côté alimentation) on chauffe les canaux dans lesquels circule le mélange gazeux, jusqu'à une température de 1200°C suivant un gradient thermique de 20°C par centimètre. Cette zone de chauffage progressif représente en général environ 20% de la longueur de la zone de chauffage. A cet effet, au moins une sonde pyrométrique 8 à thermocouple, par exemple chromel-alumel est logée dans au moins un canal 11 où circule la charge, de préférence sensiblement au niveau de la zone de chauffage où la température est maximale et transmet ses informations à un régulateur 7a qui commande par exemple un modulateur 7b à thyristor relié aux résistances électriques 5. La puissance fournie à chacune des sections de chauffage est donc modulée de façon classique en fonction de la température de chaque section.

Le mélange gazeux circule ensuite de manière continue à travers les canaux 11 qui sont au contact de la deuxième partie de la zone de chauffage où l'on maintient généralement la température à une valeur sensiblement constante et sensiblement égale à la température fixée en fin de la première zone de chauffage, c'est à dire dans le cas ci-dessus à 1200°C. A cet effet, on module, grâce à une ou plusieurs sondes 8 logées de préférence tout le long de la seconde zone de chauffage, et selon le même schéma de régulation déjà décrit ci-dessus, la puissance électrique en fonction de la température sur la longueur de la dite zone de chauffage maximum où s'effectue la majorité des réactions endothermiques, de telle façon que la variation de température autour du point de consigne soit inférieure d'environ 5°C autour de la valeur de consigne.

Cette deuxième zone de chauffage maximum correspond à environ 80 % de la zone totale de chauffage ; elle se termine par un cloisonnement étanche 14 par exemple en ciment refractaire de façon à éviter toute entrée d'effluent gazeux et/ou de fluide de refroidissement provenant de la zone suivante de refroidissement.

A la sortie de la zone de chauffage, les effluents de la réaction quittent les canaux 11 et sont refroidis dans une zone de refroidissement 10. Ils sont mis en contact avec un agent de trempe tel que du propane introduit par l'intermédiaire d'injecteurs 12 de trempe disposés à la périphérie du réacteur 1 et reliés à une source extérieure de propane (non représentée sur la figure 1). L'ensemble des gaz effluents et du propane est refroidi à une température d'environ 500 °C et recueilli par un orifice de sortie 13 à l'extrémité de la zone réactionnelle 1.

Suivant un autre mode de réalisation représentant un réacteur à trempe indirecte illustré par la figure 1B les rangées de canaux 11 où circule le mélange gazeux réactionnel s'étendent de manière continue tout le long du réacteur 1. Elles ont donc une longueur sensiblement égale à celle du réacteur.

La zone de chauffage en contact des canaux représente par exemple 60 % de la longueur totale du réacteur. Les rangées de canaux 11 d'écoulement de la charge sont tout d'abord chauffées dans leur partie supérieure (côté alimentation) dans les mêmes conditions que celles décrites ci-dessus selon la figure 1A, puis sont refroidies dans leur partie inférieure par le fluide de refroidissement qui circule dans l'espace 18 entre plaques de canaux 11, refroidissant ainsi les parois des canaux d'écoulement des effluents.

L'obturation des canaux 17, 18 par un dispositif d'obturation étanche 14 permet de délimiter et d'isoler la zone de chauffage 9 de la zone de refroidissement 10. Une autre obturation étanche 32 évite le mélange des hydrocarbures effluents avec le fluide de refroidissement.

L'arrivée du fluide de refroidissement dans cet espace, par exemple dans les rangées des canaux 18 destinés à le véhiculer peut s'effectuer sensiblement perpendiculairement à l'axe de ces rangées de canaux, au moyen d'une ouverture 16a pratiquée dans l'une des parois latérales des canaux concernés situés à la périphérie, et les canaux d'une même rangée sont en général mis en communication au niveau de l'arrivée du fluide réfrigérant par des ouvertures pratiquées dans leurs parois latérales, de façon à ce que la totalité des canaux destinés à cet usage soit parcourue par le fluide réfrigérant. Le soutirage du fluide de refroidissement peut s'effectuer aussi de manière sensiblement perpendiculaire à l'axe de ces rangées de canaux.

Le fluide de refroidissement pénètre préférentiellement dans la zone de trempe 10 par des ouvertures 16a reliées à une ligne 19, situées au voisinage du cloisonnement 14. Il circule dans les espaces 18 qui sont des canaux parallèles aux canaux 11 d'écoulement de la charge, et sensiblement de même dimension, à co-courant des effluents réactionnels, et sort du réacteur 1 par des ouvertures 16b reliées à la ligne

EP 0 289 391 B1

20.

On peut, le cas échéant, inverser le sens de circulation du fluide de refroidissement qui, dans ce cas, pénêtre dans la zone de trempe 10 par la ligne 20 et, après avoir refroidi les effluents réactionnels à contre-courant, sort de 10 par la ligne 19.

Les effluents de la réaction ainsi refroidis à une température d'environ 300 °C sont collectés à l'extrémité des canaux 11 et recueillis à l'orifice de sortie 13 par la ligne de collecte 21 comme dans la figure 1.

Si besoin est, suivant la nature des charges traitées, on peut selon un autre mode de réalisation particulier, procéder à un refroidissement en deux étapes, la première étant effectuée par trempe indirecte dans l'espace interplaques jusqu'à une température de 500–700°C, la seconde étape étant constituée par un refroidissement par injection des fluides de refroidissement dans les effluents de la zone de trempe indirecte. On peut également procéder à une trempe directe jusqu'à une température voisine de 500°C, puis refroidir les effluents vers 200°C au moyen d'un échangeur de chaleur.

On décrit ci-dessous divers modes de réalisation du réacteur pour la mise en oeuvre du procédé selon l'invention qui n'illustre que la zone de chauffage, la zone de trempe pouvant être directe ou indirecte comme décrit ci-avant selon les figures 1A et 1B.

La figure 2 représente un mode de réalisation d'un réacteur 1 vertical, de forme allongée, suivant une coupe horizontale.

Le réacteur 1 est réalisé en juxtaposant de manière non adjacente les plaques unitaires 4a, 4b, 4c... disposées verticalement, chacune d'elles contenant une rangée de canaux unitaires 11. Un ruban de conducteur électrique 5 dans un gainage isolant est disposé dans l'espace 17 entre les parois de deux plaques successives, de sorte qu'il entoure sensiblement chaque plaque 4a, 4b, 4c, définissant ainsi au moins en partie une section de chauffage. Ce peut être aussi un élément métallique résistant ou une résistance chauffante en céramique.

Des électrodes 15a et 15b permettent l'alimentation électrique de ce ruban, de chauffage de manière indépendante. Le réacteur 1 est isolé thermiquement par l'isolant 25 et est placé dans un bati. Pour minimiser les pertes d'énergie, le ruban peut adhérer aux parois de plaques voisines 4b, 4c de sorte que l'espace interplaque 17 peut être inférieur à la dimension d'un canal unitaire.

Selon un autre mode de réalisation illustré par la figure 3, l'espace 17 dans le réacteur 1 entre plaques 4 de canaux 11 réservés à la circulation de la charge est rempli par au moins une plaque sensiblement identique à la plaque 4, dont les canaux 28 sensiblement de mêmes dimensions sont disposées sensiblement perpendiculairement aux canaux 11, de sorte qu'il y a juxtaposition de plaques unitaires à canaux disposés, en alternance, verticalement et horizontalement et donc successivement en position orthogonale les uns par rapport aux autres. A l'intérieur des canaux 28 (figure 3a) sont logés des éléments métalliques résistants 27 sous forme de fils, spirales ou rubans en molybdène de manière sensiblement parallèle au plan des plaques 4. Les canaux 28 sont avantageusement remplis de poudre céramique conductrice de chaleur telle que CSi et sont obturés par un ciment céramique 31 aux extrémités desquelles dépassent les électrodes 15a et 15b. Par cette disposition, le cloisonnement aux deux extrémités de la zone de chauffage est assuré par les parois des canaux 28.

Selon un autre mode de réalisation représenté par la figure 1B, l'espace 17 entre les plaques 4 de canaux 11 est rempli par au moins une plaque sensiblement identique à la plaque 4, dont les canaux 28 sensiblement de mêmes dimensions sont sensiblement parallèles aux canaux 11. On dispose sensiblement parallèlement les unes aux autres les diverses plaques. Dans chaque plaques de canaux 28, on dispose une pluralité de résistances chauffantes en carbure de silicium sous forme de barres ou de cylindres (résistances Kanthal, marque déposée) sensiblement parallèles entre elles et dans un plan sensiblement parallèle aux plaques de façon que leur plus grande dimension soit sensiblement perpendiculaire à l'axe des canaux 11. A cet effet, les parois des canaux 28 sont creusées latéralement de façon à permettre leur positionnement adéquat dans les diverses sections de la zone de chauffage.

Les extrémités opposées de la zone de chauffage sont obstruées par des cloisonnements 14 en ciment céramique pour éviter l'entrée de la charge et du mélange d'effluent réactionnel -fluide de refroidissement dans la zone de chauffage dans le cas d'un réacteur à trempe directe et pour éviter l'entrée de la charge et du fluide de refroidissement dans le cas du réacteur à trempe indirecte.

Bien évidemment, ces modes de réalisation sont décrits à titre illustratif et non limitatif. Il est possible de changer les divers moyens de chauffage décrits ci-dessus et de les adapter aux exemples de configurations différentes de zone de chauffage. Par exemple, les résistances Kanthal pourront être utilisées dans le mode de réalisation décrit selon la figure 3, les résistances pouvant alors être soutenues au moyen d'étriers ou de supports en céramique appropriés.

Il existe, conformément à la présente invention, une forme encore améliorée de mise en oeuvre du procédé.

Selon cette forme de réalisation, on ajoute au gaz préchauffé, contenant du méthane, et constituant le réactif du procédé, l'effluent d'une torche à plasma alimentée par un gaz plasmagène. Il est en effet connu que, par passage à travers un arc électrique d'un gaz dit plasmagène, on crée un milieu réactionnel particulier, électriquement neutre mais riche en ions, électrons, atomes et/ou molécules excitées. Le gaz plasmagène peut être par exemple l'hydrogène, l'argon, la vapeur d'eau, l'azote, le méthane ou tout autre gaz courant ou le mélange de plusieurs d'entre eux en proportions variables. Ce peut être en particulier tout ou partie du gaz alimentant la zone de chauffage.

8

Selon un mode de réalisation préféré de l'invention, une partie du gaz constituant la charge peut être prélevée, avant ou après réchauffage, de préférence après réchauffage, et envoyée à la torche à plasma. L'effluent de la torche est alors injecté immédiatement au sein du reste du fluide réactif juste avant l'entrée dans la zone de pyrolyse.

On peut utiliser à cet effet de 1 à 20 % de la charge mais on peut également utiliser une source continue de gaz plasmagène. L'effet de cet ensemencement au moyen de plasma est de favoriser les étapes initiales de chauffage et donc de faciliter grandement la transformation du méthane en hydrocarbures de poids moléculaires plus élevés.

Ainsi, il devient possible de travailler à plus basse température que dans le cas où il n'y a pas d'ensemencement soit, par exemple, à une température moyenne dans le réacteur d'environ cent degrés inférieure.

Bien que le niveau thermique soit plus bas, le taux de transformation du méthane est maintenu. Par contre la tendance à faire des composés acétyléniques est réduite et la tendance à faire des composés oléfiniques et aromatiques est accrue.

Sous cette réserve, la température moyenne de chauffage se situe le plus souvent entre 600 et 1300 °C. Le but de la trempe est de ramener la température du mélange au-dessous de 400 °C, par exemple vers 100 - 350 °C ou plus bas.

Par ailleurs, on a également découvert une forme encore améliorée de mise en oeuvre du procédé.

Selon cette forme de réalisation, dont la mise en oeuvre est aisée, on ajoute au gaz préchauffé, au moins un réactif amorceur choisi dans le groupe formé par l'oxygène, l'ozone et le peroxyde d'hydrogène, dans des proportions convenables par rapport à la quantité de réactif introduite dans la zone de réaction.

Le réactif amorceur est introduit dans le mélange de gaz préchauffé, de préférence en quantité relativement faible par rapport au méthane, avant l'introduction du mélange réactionnel dans la zone de chauffage.

Sans vouloir être lié par une quelconque théorie, on peut penser que le réactif amorceur, introduit dans la charge préchauffée de préférence à au moins 300 °C et généralement de 500 à 600 °C, favorise la formation de radicaux, en particulier de radicaux méthyle, et favorise ainsi le démarrage de la réaction de conversion du méthane à plus basse température, ce qui permet d'obtenir un meilleur rendement en produits recherchés : éthylène et aromatiques.

Il n'est ainsi pas souhaitable d'avoir une quantité trop importante de réactif amorceur, car cela risquerait de conduire à la formation d'une quantité importante de radicaux et à la formation excessive de produits secondaires, en particulier d'oxydes de carbone ($CO$, $CO_2$).

La quantité du réactif amorceur, introduite dans le mélange de gaz préchauffé, exprimée en pourcentage d'atome-grammes d'oxygène introduits, par rapport à la quantité de méthane exprimée en mole, sera généralement de 0,01 et 10 % et de préférence de 0,1 à 1 %.

Selon un mode de réalisation préféré de l'invention, le réactif amorceur est l'oxygène substantiellement pur (c'est à dire renfermant moins de 1 % en volume d'impureté), ou l'oxygène dilué par un gaz inerte tel que, par exemple l'azote ou l'argon, ou encore un mélange plus complexe de gaz contenant de l'oxygène tel que par exemple l'air, ou l'air enrichi en oxygène ou l'air dilué par un gaz inerte. Il est également possible d'introduire le réactif amorceur en le diluant préalablement dans une portion de mélange gazeux que l'on veut traiter.

Lorsqu'on utilise de l'ozone celui-ci peut être utilisé sous une forme substantiellement pure ou être dilué dans un gaz comme mentionné ci-dessus pour l'oxygène. On peut par exemple employer un mélange d'oxygène et d'ozone ou de l'air ozonisé.

Lorsqu'on utilise du péroxyde d'hydrogène, celui-ci peut être utilisé sous une forme substantiellement pure ou sous une forme diluée. Il est aussi possible d'employer du peroxyde d'hydrogène aqueux, encore appelé parfois eau oxygénée, à condition que la quantité d'eau que l'on introduit alors dans le réacteur reste dans les limites précisées plus haut.

Il est possible de préchauffer le réactif amorceur avant son introduction, par exemple, jusqu'à une température de 150 °C. L'effet de l'introduction d'au moins un réactif amorceur, est de favoriser grandement la transformation du méthane en hydrocarbures de poids moléculaires élevés. Les quantités introduites étant relativement faibles, on ne forme qu'une faible proportion d'oxydes de carbone. Cette légère perte est très largement compensée par l'action bénéfique d'amorçage de la réaction, qui permet ainsi de travailler avec des températures plus basses et des temps de séjour de la charge à traiter dans la zone réactionnelle plus courts.

Ainsi, il devient dans ce cas précis généralement possible de travailler, par exemple, à une température moyenne dans le réacteur de pyrolyse d'environ soixante-dix à environ cent degrés inférieure, par rapport au cas où il n'y a pas d'introduction de réactif amorceur. Le temps de séjour dans la zone réactionnelle peut également être légèrement diminué par l'emploi d'un réactif amorceur.

## EXEMPLE

On utilise un réacteur en carbure de silicium, à trempe indirecte. La longueur de la zone réactionnelle, qui représente la longueur des canaux unitaires parcourus par la charge, est de 3 m. Le chauffage de la zone de chauffage est réalisé au moyen de résistances électriques en céramique, de marque Kanthal, qui sont intercalées entre les plaques de canaux unitaires et disposées de telle façon que leur plus grande dimension soit perpendiculaire à l'axe des canaux unitaires.

La zone de chauffage est constituée de deux parties adjacentes ; dans la première partie, longue de 0,5 m, la charge, préchauffée à 600°C, est portée à 1 200°C ; cette zone, à fort gradient thermique, 12°C/cm, est régulée thermiquement par l'intermédiaire de thermocouples disposés dans des canaux unitaires. Dans la deuxième partie, longue de 1,5 m, on maintient la charge à une température de 1 200 plus ou moins 10°C, grâce à la régulation thermique de 5 sections de chauffage constituées chacune par 5 résistances, au moyen de 5 thermocouples placés dans les canaux. La zone de chauffage est prolongée par la zone de trempe, de 1 m de longueur, qui est alimentée en air comme fluide réfrigérant. A la sortie de la zone de trempe, les effluents de la réaction sont à 250°C.

On utilise comme charge du gaz naturel, dont on a enlevé les fractions les plus lourdes (GPL) par compression et refroidissement à - 100°C ; la fraction restante, constituée essentiellement par du méthane et de l'éthane, est diluée avec de l'hydrogène pour conduire à la charge utilisée, dont la composition molaire est :

| Composé | Moles |
|---------|-------|
| $H_2$ | 100 |
| $CH_4$ | 100 |
| $C_2H_6$ | 5 |

On préchauffe ce mélange à 600°C et on procède à son craquage dans le réacteur décrit ci-dessus, en suivant les conditions opératoires décrites, avec un temps de séjour dans la zone de chauffage de 200 ms.

Après refroidissement à température ambiante, on sépare les gaz, les liquides et les solides.

Pour 205 moles du mélange utilisé, on obtient les produits suivants :

| Produits | Quantité |
|---|---|
| $H_2$ | 170,25 moles |
| $CH_4$ | 50 moles |
| $C_2H_2$ | 6,25 moles |
| $C_2H_4$ | 15,0 moles |
| Benzène | 1,5 mole |
| Phase liquide hors benzène | 104 g |
| Coke | 6 g |

**Revendications**

1. Procédé de conversion thermique de méthane en hydrocarbures de poids moléculaires plus élevés dans une zone de réaction en matière céramique comprenant une série de canaux juxtaposés groupés par rangées et s'étendant sur une partie au moins de la longueur de ladite zone de réaction parallèlement à son axe, les dites rangées de canaux étant non adjacentes les unes par rapport aux autres, la zone de réaction comprenant en outre d'une part une zone de chauffage entourant sensiblement les dites rangées de canaux soit sur la dite partie de la zone de réaction, soit sur une partie de la longueur de la dite zone de réaction quand ces canaux s'étendent sur toute la longueur de la zone de réaction, d'autre part une zone de refroidissement faisant suite à la dite zone de chauffage, caractérisé en ce que l'on fait circuler un gaz renfermant du méthane dans les canaux des dites rangées, en ce que l'on chauffe la zone de chauffage par apport d'énergie électrique par sections transversales successives, indépendantes, sensiblement perpendiculaires à l'axe de la dite zone de réaction, sensiblement parallèles au plan desdites rangées et étanches vis-à-vis des canaux de la zone de réaction, en ce que l'on introduit un fluide de refroidissement dans la dite zone de refroidissement et en ce que l'on recueille les dits hydrocarbures de poids moléculaires plus élevés à l'extrémité de la zone réactionnelle.

2. Procédé selon la revendication 1 dans lequel on chauffe une première partie de la zone de chauffage jusqu'à une température maximale au plus égale à 1500 °C de façon à réaliser un gradient thermique compris entre 5 °C/cm et 60 °C/cm sur une longueur comprise entre 5 et 50 % de la longueur totale de la zone de chauffage et dans lequel on chauffe une deuxième partie subséquente de la dite première partie de façon à ce que la variation de température tout au long de la dite deuxième partie de la zone de chauffage soit inférieure à environ 20 °C.

3. Procédé selon la revendication 1 ou 2 dans lequel la dite zone de refroidissement comprend une seconde série de canaux en alternance avec ceux où circule le gaz renfermant du méthane, et dans lequel on introduit et on soutire le dit fluide de refroidissement sensiblement perpendiculairement à l'axe des canaux de la seconde série.

4. Procédé selon la revendication 1 ou 2 dans lequel on met en contact direct le fluide de refroidisse-

11

ment avec des effluents résultant du chauffage du gaz, et on recueille les dits hydrocarbures de poids moléculaire plus élevés en mélange avec le dit fluide de refroidissement.

5. Procédé selon l'une des revendications 1 à 4 dans lequel le gaz contient en outre de l'hydrogène.

6. Dispositif pour la mise en œuvre du procédé selon l'une des revendications 1 à 5 comprenant des moyens d'alimentation (2) en mélange gazeux et des moyens d'évacuation (21) des effluents produits, caractérisé en ce qu'il comprend un réacteur (1) de forme allongée, en matière céramique, ayant un axe de symétrie et relié d'une part à une première extrémité aux dits moyens d'alimentation, et d'autre part à l'extrémité opposée aux dits moyens d'évacuation, le dit réacteur comprenant une série de canaux (11) juxtaposés, groupés par rangées, adaptés à la circulation du mélange gazeux et s'étendant sur une partie au moins de la longueur du réacteur parallèlement à son axe, les dites rangées de canaux formant une pluralité de plaques (4) non adjacentes les unes par rapport aux autres et définissant des espaces étanches (17) entre les dites plaques, le dit réacteur comportant en outre sur une première zone (côté première extrémité) des moyens de chauffage (5) électrique dans chacun des dits espaces adaptés à chauffer les dites plaques de canaux par sections transversales successives, indépendantes, sensiblement perpendiculaires à l'axe du dit réacteur et sensiblement parallèles au plan des dites plaques, les dits moyens de chauffage (5) entourant ainsi sensiblement les dites plaques de canaux soit sur la dite partie du réacteur, soit sur une partie de la longueur du dit réacteur (1) quand les dits canaux (11) s'étendent sur toute la longueur du réacteur, le dit réacteur comportant en outre des moyens d'asservissement et de modulation (7, 8) de chauffage reliés aux dits moyens de chauffage (5), le dit réacteur comportant par ailleurs sur une deuxième zone (côté extrémité opposée) contiguë la première et non communicante avec la première zone, des moyens de refroidissement (10, 12 figures 1, 18 figure 1b) des effluents adaptés à refroidir soit par circulation dans les dits espaces étanches de la deuxième zone du réacteur, les plaques (4) de canaux (trempe indirecte), soit par contact direct les effluents quittant les dits canaux (11) (trempe directe).

7. Dispositif selon la revendication 6 dans lequel la section d'un canal (11) unitaire est comprise entre 9 et 900 mm² et de préférence entre 25 et 100 mm².

8. Dispositif selon la revendication 6 ou 7 dans lequel les moyens de chauffage électrique (5) comprennent au moins un élément chauffant choisi parmi les rubans électriques isolants, les éléments métalliques résistants et les résistances chauffantes en céramique.

9. Dispositif selon la revendication 8 dans lequel l'élément chauffant est noyé dans de la céramique.

10. Dispositif selon l'une des revendications 6 à 9 dans lequel les espaces adaptés à recevoir les moyens de chauffage et de refroidissement sont compris entre 1 et 150 mm.

11. Dispositif selon l'une des revendications 6 à 10 dans lequel chacun des dits espaces (17) est rempli d'au moins une plaque de canaux (28) en matière céramique, sensiblement parallèles entre eux et disposés parallèlement aux dits canaux (11) de circulation de la charge.

12. Dispositif selon l'une des revendications 6 à 10 dans lequel chacun des dits espaces est rempli d'au moins une plaque de canaux (28) en matière céramique, sensiblement parallèles entre eux et disposés de manière sensiblement perpendiculaire aux dits canaux (11) de circulation de la charge.

13. Dispositif selon l'une des revendications 6 à 12 dans lequel les moyens (7, 8) d'asservissement et de modulation du chauffage comportent un thermocouple de commande (8), un moyen de régulation (7a) relié au dit thermocouple et un modulateur à thyristor (7b) relié au dit moyen de régulation.

14. Procédé de réalisation du dispositif selon l'une des revendications 6 à 13 caractérisé en ce que l'on juxtapose dans une première zone du réacteur, de manière sensiblement adjacente, successivement en alternance, une plaque de canaux (11) d'une série et une plaque de canaux (28) d'une deuxième série, on détermine des sections de chauffage dans la dite plaque de canaux (28) en introduisant des moyens de chauffage (5) de manière sensiblement parallèle au plan des dites plaques soit dans la direction des canaux (28) soit dans une direction sensiblement perpendiculaire aux dits canaux (28), les dits moyens de chauffage (5) étant reliés aux dits moyens d'asservissement et de modulation du chauffage (7, 8), les dites plaques de canaux (11) et (28) étant juxtaposées de telle façon que les canaux (11) de première série soient sensiblement perpendiculaires aux dites sections, on isole, si nécessaire, de la dite plaque de canaux (11) la plaque contenant les dits moyens de chauffage, on introduit dans une deuxième zone du réacteur soit un moyen de refroidissement direct (12), de préférence au voisinage de la sortie des effluents des canaux (11) soit un moyen de refroidissement indirect (19) des canaux (11) suivant lequel on juxtapose de manière alternée et sensiblement adjacente une plaque de canaux (11) adaptés à recevoir les effluents dans le prolongement des canaux (11) de la dite première zone et une plaque de canaux (18), sensiblement parallèles aux canaux (11), adaptés à faire circuler un fluide de refroidissement et l'on isole si nécessaire la dite plaque de canaux (18) par un cloisonnement (32).

## Claims

1. Process for thermally converting methane into hydrocarbons with higher molecular weights in a ceramic reaction zone with a series of juxtaposed channels grouped in rows and extending over at least a part of the total reaction zone parallel to its axis, said rows of channels being non adjacent towards one another, the reaction zone also comprising, on one hand, a heating zone which substantially surrounds said rows of channels either on said part of the reaction zone or on a part of the length of said reaction zone when said channels cover the whole length of the reaction zone, and, on the other hand, a cooling

# EP 0 289 391 B1

zone which is an extension of said heating zone, characterized in that a gas containing methane is circulated in the channels of said rows, the heating zone is heated with an electric power supply through successive, independant transverse sections which are substantially perpendicular to the axis of said reaction zone and substantially parallel to the plane of said rows and tight towards the channels of the reaction zone, a cooling fluid is introduced into said cooling zone and said hydrocarbons with higher molecular weights are collected at the end of the reaction zone.

2. A process according to claim 1, wherein a first part of the heating zone is heated up to a maximum temperature at most equal to 1500°C, in order to obtain a thermal gradient ranging from 5°C/cm to 60°C/cm on a length from 5 to 50% of the total heating zone length, and wherein a second part of the heating zone, subsequent to said first part is heated so that the temperature variation all along said second part is inferior to about 20°C.

3. A process according to Claim 1 or 2, wherein said cooling zone comprises a second series of channels alternately with those conveying the gas containing methane, and wherein said cooling fluid is introduced and withdrawn substantially perpendicularly to the axis of channels of the second series.

4. A process according to Claim 1 or 2, wherein the cooling fluid is directly contacted with effluents resulting from the heating of the gas, and said hydrocarbons with higher molecular weights are collected, mixed with said cooling fluid.

5. A process according to any one of Claims 1 to 4, wherein the gas also contains hydrogen.

6. Device for the implementation of the process according to any one of claims 1 to 5, comprising means for supplying (2) a gaseous mixture and means discharging (21) the produced effluents, characterized in that it comprises an elongate reactor (1) made of ceramic, with a symmetry axis, connected on one hand, at a first end, to said means of supply and, on the other hand, at the opposite end, to said means of discharge, said reactor containing a series of juxtaposed channels (11) grouped in rows, adapted to the circulation of the gaseous mixture and extending over at least a part of the reactor length, parallel to its axis, said rows of channels constituting a plurality of plates (4) being non adjacent to one another and defining tight spaces (17) between said plates, said reactor also containing in a first zone (first end side) means of electric heating (5) in each said space fitted for heating said plates of channels through successive, independant transverse sections, substantially perpendicular to the axis of said reactor and substantially parallel to the plane of said plates, said means of heating (5) thus substantially surrounding said plates of channels, either on said part of the reactor, or on a part of the length of said reactor (1) when said channels (11) extend over the total length of the reactor, said reactor also containing heating servo-control and modulation means (7, 8) connected to said heating means (5), said reactor containing in a second zone (opposite end side) contiguous to the first one and not communicating with the latter, means for cooling (10, 12, Fig. 1, 18; Fig. 1B) effluents adapted to cool either by circulating in said tight spaces of the second zone of the reactor the plates of channels (4) (indirect quenching) or by direct contact, the effluents leaving said channels (11) (direct quenching).

7. A device according to Claim 6, wherein the section of a unitary channel (11) ranges from 9 to 900 mm², preferably from 25 to 100 mm².

8. A device according to claim 6 or 7, wherein the electric heating means (5) comprise at least one heating element selected from the group consisting of insulating electric tapes, resisting metallic elements and ceramic heating resistances.

9. A device according to claim 8, wherein the heating element is embedded in the ceramic.

10. A device according to any one of claims 6 to 9, herein the spaces adapted to receive the heating and cooling elements range from 1 to 150 mm.

11. A device according to any one of claims 6 to 10, wherein each said space (17) is filled with at least one plate of channels (28) made of ceramic, substantially parallel to one another and placed parallel to said channels (11) conveying the gaseous mixture.

12. A device according to any one of claims 6 to 10, wherein each said space is filled with at least one plate of channels (28) made of ceramic, substantially parallel to one another and substantially perpendicular to said channels (11) conveying the gaseous mixture.

13. A device according any one of claims 6 to 12, wherein the heating servo-control and modulation means (7, 8) comprise a control thermocouple (8), a regulation means (7a) connected to said thermocouple and a thyristor modulator (7b) connected to said regulation means.

14. A process for realizing the device according to any one of claims 6 to 13, characterized in that a plate of channels (11) of one series and a plate of channels (28) of second series are juxtaposed in a first reactor zone, in a substantially adjacent way and alternately, heating sections are determined in said plate of channels (28) by introducing heating means (5) substantially parallel to the plane of said plate, either in the direction of the channels (28) or in a direction substantially perpendicular it said channels (28), said heating means (5) being connected to said heating servo-control and modulation means (7, 8), said plates of channels (11) and (28) being juxtaposed so that channels (11) of the first series are substantially perpendicular to said sections, the plate containing said heating means is insulated, if need be, from saids plate of channels (11) and characterized in that into a second reactor zone either a direct cooling means (12), preferably close to the outlet of the effluents of channels (11), or an indirect cooling means (19) of channels (11) is introduced, following which a plate of channels (11) adapted to receive the effluents in the extension of channels (11) of said first zone is alternately juxtaposed, in a substantially adjacent way, with a plate of channels (18) substantially parallel to channels (11), adapted for circulating a cooling fluid and in that, if need be, said plate of channels (18) is insulated with a partitioning (32).

**Patentansprüche**

1. Verfahren zur thermischen Umwandlung von Methan in Kohlenwasserstoffe höheren Molekulargewichtes in einer Reaktionszone aus keramischem Material mit einer Reihe nebeneinander angeordneter Kanäle, die gruppenweise angeordnet sind und sich wenigstens über einen Teil der Länge dieser Reaktionszone parallel zu ihrer Achse erstrecken, wobei diese Gruppe von Kanälen einander nicht benachbart sind und die Reaktionszone im übrigen einerseits eine Heizzone umfaßt, die im wesentlichen diese Reihen von Kanälen entweder auf diesen Teil der Reaktionszone oder auf einem Teil der Länge dieser Reaktionszone umgibt, wenn die Kanäle sich über die gesamte Länge der Reaktionszone erstrecken und andererseits eine Kühlzone umfaßt, die sich an diese Heizzone anschließt, dadurch ggekennzeichnet, daß man ein methanenthaltendes Gas in den Kanälen dieser Gruppen zirkulieren läßt, daß man die Heizzone durch Zuführung elektrischer Energie über aufeinanderfolgende unabhängige transversale Abschnitte im wesentlichen senkrecht zur Achse dieser Reaktionszone, die im wesentlichen parallel zur Ebene dieser Gruppen und dicht gegenüber Kanälen der Reaktionszone sind, wärmt, daß man ein Kühlfluid in diese Kühlzone einführt und daß man diese Kohlenwasserstoffe erhöhten Molekulargewichts am Ende der Reaktionszone sammelt.

2. Verfahren nach Anspruch 1, dadurch gekennzeichnet, daß man einen ersten Teil der Heizzone bis auf eine Maximaltemperatur von höchstens 1500°C derart erhitzt, daß ein Wärmegradient zwischen 5°C/cm und 60°C/cm über eine Länge zwischen 5 und 50% der Gesamtlänge der Heizzone realisiert wird und daß man eine zweite Zone anschließend an diesen ersten Teil derart erwärmt, daß die Temperaturveränderung längs des gesamten zweiten Teils der Heizzone kleiner als 20°C wird.

3. Verfahren nach Anspruch 1 oder 2, dadurch gekennzeichnet, daß diese Kühlzone eine zweite Reihe von Kanälen aufweist, die mit denjenigen abwechseln, in denen das methanenthaltende Gas zirkuliert und daß man dieses Kühlfluid im wesentlichen senkrecht zur Achse der Kanäle der zweiten Reihe einführt und abzieht.

4. Verfahren nach Anspruch 1 oder 2, dadurch gekennzeichnet, daß man das Kühlfluid direkt mit den aus der Erwärmung der Gases resultierenden Abströmen kontaktiert und diese Kohlenwasserstoffe erhöhten Molekulargewichts im Gemisch mit diesem Kühlfluid sammelt.

5. Verfahren nach einem der Ansprüche 1 bis 4, dadurch gekennzeichnet, daß das Gas im übrigen Wasserstoff enthält.

6. Vorrichtung zur Durchführung des Verfahrens nach einem der Ansprüche 1 bis 5 mit Mitteln zum Speisen (2) mit gasförmigem Gemisch und Mitteln zum Abziehen (21) der Abströmprodukte, dadurch gekennzeichnet, daß sie einen Reaktor (1) länglicher Form aus keramischem Material mit Symmetrieachse umfaßt, der einerseits an einem ersten Ende mit diesen Speiseeinrichtungen und andererseits an dem gegenüberliegenden Ende mit diesen Abzugseinrichtungen verbunden ist, wobei der Reaktor eine Reihe von nebeneinander angeordneten in Gruppen zusammengefaßten Kanälen (11) umfaßt, die für die Zirkulation des gasförmigen Gemisches ausgelegt sind und sich wenigstens über einen Teil der Länge des Reaktors parallel zu seiner Achse erstrecken, wobei diese Gruppen von Kanälen eine Vielzahl von Platten (4) bilden, die einander nicht benachbart sind und dichte Räume (17) zwischen diesen Platten bilden, wobei der Reaktor im übrigen in einer ersten Zone (auf der Seite des ersten Endes) elektrische Heizmittel (5) in jedem dieser Räume umfaßt, die so ausgebildet sind, daß sie diese Platten von Kanälen durch aufeinanderfolgende Abschnitte wärmen, die unabhängig und im wesentlichen senkrecht zur Achse dieses Reaktors angeordnet sind und im wesentlichen parallel zur Ebene dieser Platten verlaufen, wobei diese Heizmittel (5) so im wesentlichen die Platten der Kanäle umschließen, entweder auf diesen Teil des Reaktors oder auf einem Teil der Länge des Reaktors (1) gesehen, wenn diese Kanäle (11) sich über die gesamte Länge des Reaktors erstrecken, wobei der Reaktor im übrigen Heizhilfssteuer- und Heizmodulationsmittel (7, 8) umfaßt, die mit diesen Heizmitteln (5) verbunden sind, wobei der Reaktor im übrigen in einer zweiten Zone (Seite des gegenüberliegenden Endes) angrenzend an diese erste, jedoch mit ihr nicht in Verbindung stehende Kühlmittel (10, 12 Fig. 1, 18 Fig. 1b) der Abströme umfaßt, die so ausgebildet sind, daß sie entweder durch Zirkulation in diesen dichten Räumen der zweiten Zone des Reaktors die Platten (4) der Kanäle (indirektes Abschrecken) oder durch direkten Kontakt der Abströme beim Verlassen der Kanäle (11) kühlen (direkte Abschreckung).

7. Vorrichtung nach Anspruch 6, dadurch gekennzeichnet , daß der Querschnitt eines Einheitskanals (11) zwischen 9 und 900 mm² und vorzugsweise zwischen 25 und 100 mm² beträgt.

8. Vorrichtung nach Anspruch 6 oder 7, dadurch gekennzeichnet, daß die elektrischen Heizmittel (5) wenigstens ein Heizelement umfassen, das gewählt ist aus isolierenden elektrischen Bändern, metallischen Widerstandselementen und heizenden Widerständen aus Keramik.

9. Vorrichtung nach Anspruch 8, dadurch gekennzeichnet, daß das Heizelement in die Keramik eintaucht.

10. Vorrichtung nach einem der Ansprüche 6 bis 9, dadurch gekennzeichnet, daß die Räume, die so ausgebildet sind, daß sie die Heizmittel und Kühlmittel aufnehmen, zwischen 1 und 150 mm betragen.

11. Vorrichtung nach einem der Ansprüche 6 bis 10, dadurch gekennzeichnet, daß jeder dieser Räume (17) mit wenigstens einer Platte aus Kanälen (28) aus keramischem, Material gefüllt ist, die im wesentlichen parallel zueinander laufen und parallel zu diesen Zirkulationskanälen (11) für die Charge angeordnet sind.

12. Vorrichtung nach einem der Ansprüche 6 bis 10, dadurch gekennzeichnet, daß jeder dieser Räume mit wenigstens einer Platte von Kanälen (28) aus keramischem Material gefüllt ist, die im wesentlichen parallel zueinander und damit im wesentlichen senkrecht zu diesen Kanälen (11) für die Zirkulation der Charge verlaufen.

13. Vorrichtung nach einem der Ansprüche 6 bis 12, dadurch gekennzeichnet, daß die Heizhilfssteuer- und Modulationsmittel (7, 8) ein Steuerthermoelement (8), ein Stellmittel (7a), das mit dem Thermoelement verbunden ist, sowie einen Modulator mit Thyristor (7b) umfassen, der mit diesem Steuermittel verbunden ist.

14. Verfahren zur Herstellung der Vorrichtung nach einem der Ansprüche 6 bis 13, dadurch gekennzeichnet, daß man nebeneinander in einer ersten Zone des Reaktors im wesentlichen angrenzend nacheinander abwechselnd eine Platte mit Kanälen (11) einer Reihe und eine Platte mit Kanälen (28) einer zweiten Reihe anordnet, man Heizquerschnitte in dieser Platte von Kanälen (28) bestimmt, indem man Heizmittel (5) im wesentlichen parallel zur Ebene dieser Platten entweder in der Richtung der Kanäle (28) oder in einer Richtung im wesentlichen senkrecht zu diesen Kanälen (28) einführt, wobei die Heizmittel (5) mit diesen Heizhilfssteuer- und Modulationsmitteln (7, 8) verbunden sind, wobei diese Platten von Kanälen (11) und (28) derart nebeneinander angeordnet sind, daß die Kanäle (11) der ersten Reihe im wesentlichen senkrecht zu diesen Abschnitten verlaufen, man gegebenenfalls gegen die Platte von Kanälen (11) die Platte isoliert, welche die Heizmittel enthält, man in eine zweite Zone des Reaktors entweder ein Mittel zur direkten Kühlung (12), vorzugsweise benachbart dem Austritt der Abströme der Kanäle (11) oder ein Mittel zur indirekten Kühlung (19) der Kanäle (11), einführt, wonach man abwechselnd und im wesentlichen benachbart eine Platte aus Kanälen (11) die so ausgelegt sind, daß sie die Abströme in der Verlängerung der Kanäle (11) aus dieser ersten Zone empfangen und eine zweite Platte von Kanälen (18) anordnet, die im wesentlichen parallel zu den Kanälen (11) so ausgelegt sind, daß sie ein Kühlfluid zirkulieren lassen und daß man gegebenenfalls diese Platte von Kanälen (18) durch eine Trennwand (32) isoliert.

FIG.1A

FIG.1B

FIG.2

FIG.3

FIG.3A